# EUROPEAN PATENT SPECIFICATION

(11) **EP 2 881 469 B1**
(45) Date of publication and mention of the grant of the patent: **05.04.2017**
(21) Application number: 13195578.3
(22) Date of filing: 03.12.2013
(51) Int. Cl.: C12N 15/74, C12R 1/01

(54) **Shuttle vectors and expression vectors for Amycolatopsis**
Shuttlevektoren und Expressionsvektoren für Amycolatopsis
Shuttle-vecteurs et expression-vecteurs pour Amycolatopsis

(43) Date of publication of application: 10.06.2015
(73) Proprietor: Symrise AG, 37603 Holzminden (DE)
(72) Inventor: Steinbüchel, Alexander, 48341 Altenberge (DE); Fleige, Christian, 59075 Hamm (DE); Lambrecht, Stefan, 37619 Hehlen (DE); Pesaro, Manuel, 37688 Wehrden (DE)
(74) Representative: Fabry, Bernd

(56) References cited:
- WO-A1-2012/172108
- H. PRIEFERT ET AL: "Transformation of the Pseudonocardiaceae Amycolatopsis sp. strain HR167 is highly dependent on the physiological state of the cells", APPLIED MICROBIOLOGY AND BIOTECHNOLOGY, vol. 58, no. 4, 1 March 2002 (2002-03-01), pages 454-460, XP055106962, ISSN: 0175-7598, DOI: 10.1007/s00253-001-0920-5
- C. FLEIGE ET AL: "Investigation of the Amycolatopsis sp. Strain ATCC 39116 Vanillin Dehydrogenase and Its Impact on the Biotechnical Production of Vanillin", APPLIED AND ENVIRONMENTAL MICROBIOLOGY, vol. 79, no. 1, 12 October 2012 (2012-10-12), pages 81-90, XP055106545, ISSN: 0099-2240, DOI: 10.1128/AEM.02358-12
- CHRISTIAN FLEIGE ET AL: "Construction of expression vectors for metabolic engineering of the vanillin-producing actinomycete Amycolatopsis sp. ATCC 39116", APPLIED MICROBIOLOGY AND BIOTECHNOLOGY, vol. 98, no. 14, 18 April 2014 (2014-04-18), pages 6387-6395, XP055148582, ISSN: 0175-7598, DOI: 10.1007/s00253-014-5724-5

## Description

### FIELD OF INVENTION

The present invention belongs to the area of genetic engineering and covers new shuttle and expression vectors for *Amycolatopsis* sp. ATCC 39116, processes for obtaining them and their application, particular in the production of nature-identic vanillin.

### STATE OF THE ART

The tremendous progress in the development of DNA sequencing and manipulation techniques, which were established in the past decades, stimulated the targeted design of microbial production strains for various applications. One prominent example is the production of amino acids, such as L-lysine or L-glutamate by *Corynebacterium glutamicum* [2].

Economic interests as well as the increasing demand for sustainable, bio-based products, require the improvement of fermentative production processes, which often have to compete with cheaper chemical approaches. Apart from the optimization of the fermentative process by chemical engineering, the application of metabolic engineering for the generation of improved production strains provides the largest potential to develop profitable processes [20].

The actinomycete *Amycolatopsis* sp. ATCC 39116 is used by the industry for the fermentative production of vanillin from ferulic acid, which is a cell wall component of higher plants [15]. In this case the produced vanillin can be labelled as 'natural' since it is extracted from natural sources or "obtained by appropriate physical, enzymatic or microbiological processes from material of vegetable, animal or microbiological origin" (European Commission (EC) Regulation No 1334/2008) [9]. Since natural vanillin has a remarkably higher market price in comparison to the chemically synthesized 'nature-identical' version (up to 1000-fold), this approach can compete with the cheaper chemical processes based on petrochemicals or lignin derivatives [6].

In order to stop the catabolism of the produced vanillin and therefore to achieve higher vanillin yields and concentrations in production, first attempts were made to engineer the metabolism of *Amycolatopsis* sp. ATCC 39116 and related bacteria [8, 10, 18, 21]. To gain further access to an efficient genetic engineering of *Amycolatopsis* sp. ATCC 39116, new molecular tools have to be developed.

Overexpression of native or heterologous genes in microorganisms can lead to a significant improvement of biotechnological production strains. Beside the systematic deletion or down-regulation of genes, which are involved in catabolic or competing pathways of the desired product, the controlled expression of anabolism related genes is also important to introduce rational changes in the genetic background of the used organism [20].

The capability to overexpress native or foreign genes depends on the availability of host-vector systems such as plasmids, genetic elements like functional promoters and efficient transformation protocols. Although some techniques can be adopted from phylogenetically related bacteria, each part has to be developed individually for a particular bacterial strain [23].

Actinomycetes produce a remarkable amount of secondary metabolites that are industrially used in fields like agriculture, food production and especially pharmacology [17]. Thus, they have been intensively studied aiming at higher titers of the compound of interest or at producing new metabolites with different bioactive functions. Within the group of actinobacteria research on engineering of *Streptomyces* genomes is noteworthy. Besides the commonly used *E. coli* systems, heterologous expression in *Streptomyces* is well established and often used for genes, which are of actinobacterial origin. However, most systems are of limited utility for other Actinomycetes such as *Nocardia or Amycolatopsis.*

To make *Amycolatopsis* sp. HR167, whose 16S-rDNA-sequence is identical to that of *Amycolatopsis* sp. ATCC 39116, accessible for genetic engineering. The generation of pRLE6, an *E. coli-Amycolatopsis* shuttle vector based on the origin of replication of the indigenous plasmid pA387 from *Amycolatopsis benzoatilytica* DSM 43387 is known from the state of the art {14]. Plasmid pRLE6 is a religated EcoRI-fragment of the *Amycolatopsis mediterranei* shuttle vector pRL60 lacking the erythromycin resistance- and the α-amylase gene. The vector also replicates autonomously in *Amycolatopsis* sp. ATCC 39116 without further deletions or rearrangement. However, the vector did not harbour a conventional multiple cloning site with an integrated promoter, allowing the expression of a cloned gene.

Due to the lack of an integrated promoter and missing information about the functionality of these genetic elements in *Amycolatopsis,* the access to genetic engineering was limited so far.

Therefore, the object of the present invention has been the construction and application of a plasmid-based gene expression system for *Amycolatopsis* sp. ATCC 39116 that allows efficient genetic engineering of this industrially relevant production strain.

### DESCRIPTION OF THE INVENTION

A first object of the present invention is related to a new shuttle vector (p6apra) with resistance to apramycin obtainable in that shuttle vector pRLE6 of *E.coli-Amycolatopsis*
(a) is subjected to digestion with a suitable endonuclease (SmaI*,* XmaI) to remove the *npt* gene from said vector or the vector backbone is amplified by inverse PCR without the *npt gene* and
(b) the apramycin resistance gene *aac(3)VI* is inserted into the vector backbone by ligation,
and a corresponding process for obtaining shuttle vector p6apra, wherein said shuttle vector pRLE6 of *E.coli-Amylocatopsis*
(a) is subjected to digestion with Smal to remove the *npt* gene from said vector and
(b) the apramycin resistance cassette of the transposon pMA5096 is inserted into the vector backbone by blunt end ligation.

In addition, further embodiments of the present invention concern expression vectors as defined above obtained by derivatisation ("embodiments") and the corresponding processes. In detail:
(I) A first embodiment, wherein
   (a) *lac*-promoter with the multiple cloning site of the plasmid pBluescript SK- is amplified via PCR
   (b) the PCR product is cloned into the EcoRV site of the p6apra vector according to Claim 1.
   and a corresponding process for obtaining a first expression vector, wherein
   (a) *lac*-promoter with the multiple cloning site of the plasmid pBluescript SK- is amplified via PCR
   (b) the PCR product is cloned into the EcoRV site of the p6apra vector according to Claim 1.
(II) A second embodiment, wherein
   (a) a 395-bp fragment of the genome of *Amycolatopsis* sp. ATCC 39116 upstream of the start codon of *vdh* (GTG) containing a promoter sequence is amplified via PCR,
   (b) a NcoI-site (CCATGG) is inserted into the reverse primer 6bp downstream of the Shine-Dalgarno sequence of *vdh* (AGGAG), and
   (c) the PCR product is cloned into the EcoRV site of the p6apra vector according to Claim 1.
   and a corresponding process for obtaining a second promoted shuttle vector, wherein
   (a) a 395-bp fragment of the genome of *Amycolatopsis* sp. ATCC 39116 upstream of the start codon of *vdh* (GTG) containing a promoter sequence is amplified via PCR,
   (b) a Ncol-site (CCATGG) is inserted into the reverse primer 6bp downstream of the Shine-Dalgarno sequence of *vdh* (AGGAG), and
   (c) the PCR product is cloned into the EcoRV site of the p6apra vector according to Claim 1.
(III) A third embodiment, wherein
   (a) the promoter sequence of the kanamycin resistance gene (Km^{r}) is amplified via PCR, (b) a Ncol-site (CCATGG) is inserted into the reverse primer 9bp downstream of the Shine-Dalgarno sequence of *npt,* and
   (b) the PCR product is cloned into the EcoRV site of the p6apra vector according to Claim 1.
   and a corresponding process for obtaining a third expression vector, wherein
   (a) the promoter sequence of the kanamycin resistance gene (Km^{r}) is amplified via PCR, (b) a Ncol-site (CCATGG) is inserted into the reverse primer 9bp downstream of the Shine-Dalgarno sequence of *npt,* and
   (b) the PCR product is cloned into the EcoRV site of the p6apra vector according to Claim 1.
(IV) A fourth embodiment, wherein
   (a) *ermE** promoter from *Saccharopolyspora erythraea* is removed from the plasmid pIAGO, and
   (b) the fragment is cloned into the EcoRV site of the p6apra vector according to Claim 1.
   and a process for obtaining a fourth expression vector, wherein
   (a) *ermE** promoter from *Saccharopolyspora erythraea* is removed from the plasmid pIAGO, and
   (b) the fragment is cloned into the EcoRV site of the p6apra vector according to Claim 1.

Surprisingly, it has been observed that the new expression vectors, obtained by inserting different promoter elements, overcome the drawbacks reported in the state of the art.

### More detailed description of the invention

In a first step a pRLE6 derivative was constructed that conferred apramycin resistance instead of kanamycin. This was necessary, since it was observed that recombination events between the episomal and an inactive genomic copy of the kanamycin resistance gene *npt* occurs. Furthermore, a kanamycin resistant deletion mutant of *Amycolatopsis* sp. ATCC 39116 was used. The new transformation vector p6apra was also maintained without further modification under the selection of apramycin. Applicant was able to isolate plasmid-DNA from each tested clone. Therefore, the plasmid replicated autonomously from the genome and did not integrate. This was not the fact, if pRLE6 was used for the transformation of *Amycolatopsis* sp. ATCC 39116.

In order to generate a multiple cloning site like in conventional expression vectors, four different promoter sequences were inserted into the shuttle vector to enable the transcription of colinear cloned genes. After integration of each promoter, at least six unique restriction sites were obtained which could be used for cloning, respectively. In case of the two promoters, which were known to be functional in *Amycolatopsis* sp. ATCC 39116, the *vdh* and Km^{r} promoter, their genetic organization was successfully maintained by introducing an additional Ncol-site. This restriction site allowed cloning the ATG start codon of a gene of interest six or nine base pairs downstream of the respective Shine-Dalgarno-sequence. As a third promoter the *lac*-promoter was chosen, which is useful to enable heterologous expression in a broad range of bacteria. Finally the *ermE**-promoter was inserted, in order to achieve a strong constitutive expression in different actinomycetes [3].

In order to determine the functionality of the cloned promoters, the glucuronidase gene *gusA* was inserted into the respective p6apra derivatives. *gusA was chosen* as a reporter gene, since the activity of the encoded enzyme GUS can easily be detected directly on agar plates. Additionally, the activity can be assayed in a very simple and cheap but sensitive enzyme test [16]. Finally, *Amycolatopsis* sp. ATCC 39116 did not exhibit any inherent glucuronidase activity.

As a main requirement for the successful establishment of a plasmid-based expression system for *Amycolatopsis* sp. ATCC 39116 the stable replication of the new expression vectors was observed. All generated plasmids did not undergo any modification as it was previously observed, for *Amycolatopsis* sp. HR167 and other *Amycolatopsis* strains [14, 23].

Following the analysis of the genetic stability of the new vector system the functionality of the integrated promoters was analysed by using the reporter gene *gusA.* Since *Amycolatopsis* sp. ATCC 39116 does not exhibit an endogenous glucuronidase activity, the hydrolysis of the substrates X-Gluc and *p*-nitrophenyl-ß-D-glucuronide can directly be correlated to the expression of GUS encoded by the reporter gene. Furthermore, GUS does not need any cofactors, and the activity is therefore only limited to the amount of enzyme [16].

Surprisingly, the transcription of *gusA* mediated by the *ermE**-promoter led to the highest glucuronidase activity. This was proven within both the qualitative assay on agar plates as well as the quantitative enzyme assay.

Gene expression activated by *PermE** led to a six-fold higher GUS-activity in comparison to the Km^{r} promoter, which is the second strongest. Furthermore, the glucuronidase activity in the soluble fraction of *Amycolatopsis* sp. ATCC 39116 p6permE::*gusA* was 75-fold higher than the activity of *Amycolatopsis* sp. ATCC 39116 p6pvdh::*gusA* and even 7550-fold higher than the activity *Amycolatopsis* sp. ATCC 39116 p6plac::*gusA*. The lower expression under the control of *lacP* is remarkable, due to the fact, that the promoter is commonly used in many different actinomycetes, including strains of *Nocardia, Gordonia* or *Rhodococcus.* Interestingly, the *lac*-promoter was also used in previous studies with *Amycolatopsis* sp. HR167 to enable the heterologous expression of foreign genes [18]. In this case the heterologous gene was cloned into pBluescript SK- under the control of *lacP,* and in a next step the whole plasmid was ligated with pRLE6.

The differences in the expression activation were also observable in SDS-PAGE analysis, which revealed a prominent protein signal with an apparent molecular weight of 69 ± 2 kDa for *Amycolatopsis* sp. ATCC 39116 p6permE::*gusA*. This band was missing in the sample of the control strain and was only very weak in the samples of the strains containing plasmids possessing the *lacP* or the *vdh* promoter. The apparent molecular weight was also in good agreement with the postulated size of the β-glucuronidase which is 68.2 kDa [12].

The new plasmids provide new capabilities for the expression of heterologous genes in *Amycolatopsis* sp. ATCC 39116. Especially the possibility to choose between different promoters, providing remarkable differences in the expression strength, makes genetic engineering of the industrially relevant actinomycete *Amycolatopsis* sp. ATCC 39116 more easy and efficient.

### Bacterial strains, plasmids and cultivation conditions

All bacterial strains and plasmids used in this study are listed in Table 1. Cells of *Escherichia coli* were grown in Lysogeny Broth (LB) medium at 37 °C (Sambrook et al. 1989). Cells of *Amycolatopsis* sp. ATCC 39116 were grown at 45 °C in Caso medium (Merck, Darmstadt, Germany). For selection of plasmid harboring strains, antibiotics were added to the medium as follows: Kanamycin: 50 µg/ml for *E. coli;* Apramycin: 50 µg/ml for *E. coli* and *Amycolatopsis.* Cell growth was measured by determination of the optical density at 400 nm (*Amycolatopsis* sp. ATCC 39116) or 600 nm (*E. coli*)*.*

### DNA isolation and manipulation

Plasmid DNA was isolated from *E. coli* using the "peqGOLD Plasmid MiniPrep Kit I" (PEQLAB GmbH, Erlangen, Germany). Plasmid isolation from *Amycolatopsis* sp. ATCC 39116 was performed using the "Qiagen Plasmid Purification Kit (Mini)" (Qiagen GmbH, Hilden, Germany). Upon plasmid isolation from *Amycolatopsis* sp. ATCC 39116, the DNA was transferred to *E. coli* again, to yield a sufficient amount that could be further analyzed. DNA was digested by restriction endonucleases (Thermo Fisher Scientific GmbH, Schwerte, Germany) under the conditions described by the manufacturer. Phusion Hot-Start II high-fidelity DNA polymerase, T4 DNA ligase and T4 DNA polymerase (Thermo Fisher Scientific GmbH, Schwerte, Germany) were used according to the instructions of the manufacturer. Oligonucleotides were purchased from Eurofins MWG Synthesis GmbH (Ebersberg, Germany). DNA fragments were isolated from agarose gels or reaction mixtures using the "peqGOLD Gel Extraction Kit" (PEQLAB GmbH, Erlangen, Germany).

### Transfer of DNA

Plasmids were transferred into *E. coli* by employing the CaCl₂-method (Sambrook et al. 1989), whereas the transfer of plasmids into *Amycolatopsis* sp. ATCC 39116 was performed by direct mycelia transformation as previously described.

### Plasmid construction

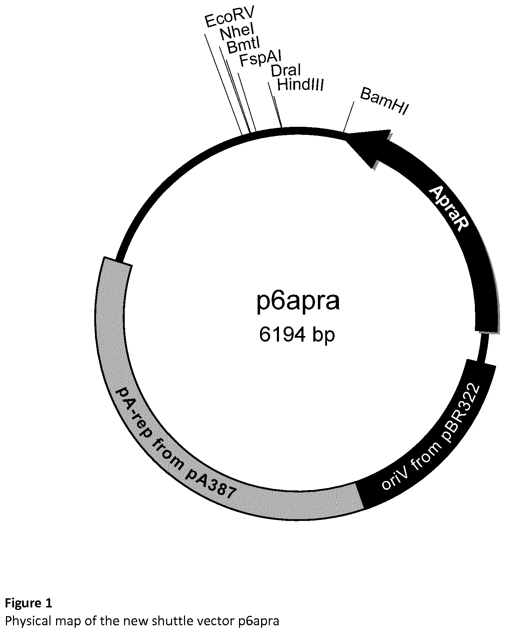

The kanamycin resistance gene from pRLE6 [23] was replaced by the apramycin resistance gene from pMA5096 [1] to generate p6apra. For this purpose pRLE6 was digested with Smal to release a 1006-bp fragment containing the whole coding sequence of *npt.* The apramycin resistance gene *aac(3)VI* was amplified by PCR using the Phusion Hot-Start II high-fidelity DNA polymerase and the oligonucleotides Apra_for and Apra_rev (**Table 1**). The amplified product was subsequently ligated with the Smal-digested vector backbone of pRLE6. After transformation of *E. coli* Mach-1 T1, the resulting plasmid p6apra was isolated and sequenced. The vector provides a multiple cloning site consisting of seven unique restriction sites (EcoRV, NheI, Bmtl, FspAI, DraI, HindIII, BamHI, see also **Figure 1**). After transformation of *E. coli* ER 2925 to yield Dam- and Dcm-unmethylated DNA, p6apra was transferred to *Amycolatopsis* sp. ATCC 39116 and resulting mutants were analyzed for a stable replication of the new plasmid.

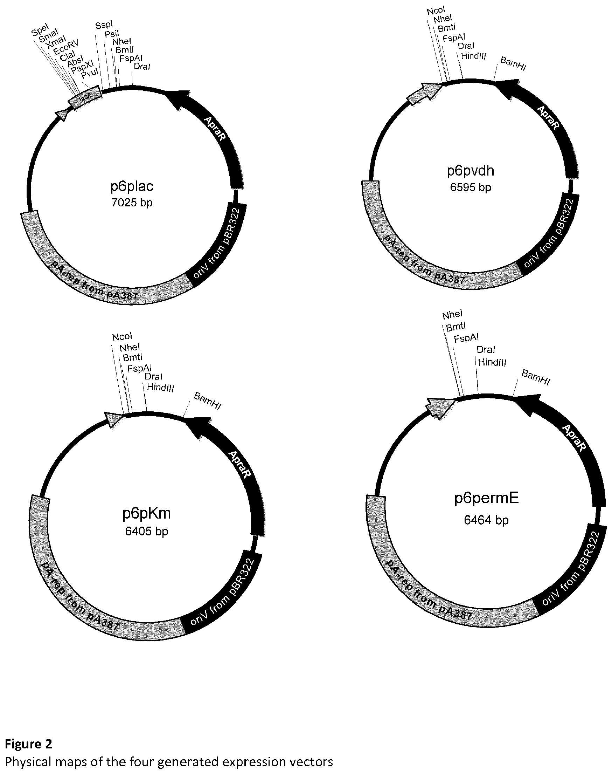

To make the transformation vector suitable for heterologous expression in *Amycolatopsis* sp. ATCC 39116 four different promoters were integrated into the single EcoRV-site of p6apra as shown in **Figure 2****.**

At first the whole multiple cloning site of pSK- including *lacZ* was amplified using the oligonucleotides pSK_MCS_for and pSK_MCS_rev (**Table 1**) and Phusion high-fidelity DNA polymerase. Furthermore, the promoter region of the kanamycin resistance gene of pRLE6 was amplified using the oligonucleotides pKm_for and pKm_Ncol_rev (**Table 1**). The reverse primer includes a Ncol-site (CCATGG) which allows to clone the start codon (ATG) of a gene of interest 9 bp downstream of the included Shine-Dalgarno-sequence of *npt.* Additionally the upstream region of *vdh* [10] was amplified using the oligonucleotides pvdh_for and pvdh_Ncol_rev (**Table 1**). The ATG within the Ncol-site of the reverse primer is located 6 bp downstream of the Shine-Dalgarno-sequence which was designed analogous to the genetic organization of the *vdh* open reading frame in the genome of *Amycolatopsis* sp. ATCC 39116.

The *ermE**-promoter was isolated from pIAGO [22] by digestion with KpnI/BamHI. The fragment was purified from an agarose gel and blunt ends were generated using T4 DNA polymerase.

Each of the four promoter fragments was cloned into the EcoRV site of p6apra via blunt end ligation. The resulting plasmids were transferred to *E. coli* Mach-1 T1, isolated, and subsequently sequenced to analyze the correct integration and orientation of the promoter fragments colinear to the residual multiple cloning site.

In order to investigate the functionality and expression strength of the cloned promoters, the glucuronidase gene *gusA* was isolated as Spel/EcoRV fragment from pSETGUS (Myronovskyi et al. 2011) and was subsequently cloned into the Nhel/Dral-linearized expression vectors (p6pKm, p6pvdh, p6permE) colinear to the integrated promoters. For p6plac, *gusA* was cloned into the SpeI/EcoRV sites provided by the additionally cloned MCS.

After transformation of *E. coli* ER 2925, the expression vectors were transferred to *Amycolatopsis* sp. ATCC 39116, and the resulting strains were analyzed for a stable replication of the new plasmids.

### Preparation of crude cell extracts and soluble fractions

Cell pellets of *Amycolatopsis* sp. ATCC 39116 were resuspended in 3 to 4 ml of 50 mM potassium phosphate buffer (pH 7.0). Crude extracts were obtained by three passages through a French pressure cell (120 MPa). For the preparation of soluble fractions, cell debris was removed by centrifugation for 60 min at 20.000 x g at 4 °C. For subsequent enzyme assays, samples were stored on ice.

### Glucuronidase enzyme assay

The spectrophotometric measurement of glucuronidase activity in cell lysates was accomplished as described before. The reaction was initiated by the addition of p-nitrophenyl-ß-D-glucuronide. Formation of p-nitrophenol was observed at 415 nm (ε = 7.76 cm²/µmol). Enzyme activity is stated in units (U). One unit is defined as the amount of enzyme which generates 1 µmol *p*-nitrophenol per min.

To detect β-glucuronidase activity on agar plates, the colonies were overlaid with 1ml of 1mM 5-bromo-4-chloro-3-indolyl-beta-D-glucuronic acid (X-Gluc) solution. After a short incubation colonies containing hydrolytic GUS activity turned blue because of the formation of 5,5'-dibromo-4,4'-dichloro indigo.

### Polyacrylamide gel electrophoresis

Samples of the soluble fractions were analyzed for their protein contents according to the method of Bradford (1976). Sodium dodecyl sulfate polyacrylamide gel electrophoresis (SDS-PAGE) was performed in 12.5% (wt/vol) gels as described by [13]. Amounts of proteins were added as indicated in the text. Staining of proteins was performed with Serva Blue G [28]. The following molecular weight reference proteins were purchased from Thermo Fisher Scientific GmbH (Schwerte, Germany): "PageRuler Prestained Protein Ladder" and "Unstained Protein Molecular Weight Marker".

### INDUSTRIAL APPLICATION

Another object of the present invention refers to plasmid expression system for *Amycolatopsis* sp. ATCC 39116 comprising at least one of the expression vectors according to any of the Claims 3, 5, 7 or 9.

Further on, the invention encompasses the use of at least one of the expression vectors (I) to (IV) for replication in *Amycolatopsis* sp. ATCC 39116 and for heterologous expression of genes in *Amycolatopsis* sp. ATCC 39116 respectively.

Also encompassed by the present invention is the use of *Amycolaptopis* sp. ATCC 39116 containing at least one of the expression vectors (I) to (IV) in a process for the manufacture of vanillin.

Finally, the invention also covers a process for making nature-identic vanillin by fermentation of ferulic acid by means of microorganisms selected from *Amycolaptopis* sp. ATCC 39116 containing at least one the expression vectors (I) to (IV)

### RELEVANT LITERATURE

The following documents represent the relevant state of the art. The numbers in the specification refer to these documents.
*[1]* Banh Q, Arenskötter M, Steinbüchel A Establishment of Tn5096-based transposon mutagenesis in Gordonia polyisoprenivorans. Appl Environ Microbiol 71:5077-5084 (2005**)**
[2] Becker J, Wittmann C Systems and synthetic metabolic engineering for amino acid production - the heartbeat of industrial strain development. Curr Opin Biotechnol 23:718-726 (2012**)**
[3] Bibb MJ, Janssen GR, Ward JM Cloning and analysis of the promoter region of the erythromycin resistance gene (ermE) of Streptomyces erythraeus. Gene 38:215-226 (1985**)**
[4] Bibb MJ, White J, Ward JM, Janssen GR The mRNA for the 23S rRNA methylase encoded by the ermE gene of Saccharopolyspora erythraea is translated in the absence of a conventional ribosome-binding site. Mol Microbiol 14:533-545 (1994**)**
[5] Bradford MM A rapid and sensitive method for the quantitation of microgram quantities of protein utilizing the principle of protein dye binding. Anal Biochem 72:248-254 (1976**)**
[6] Converti A, Aliakbarian B, Domínguez JM, Vázquez GB, Perego P Microbial production of biovanillin. Brazilian J Microbiol 41:519-530 (2010**)**
[7] Davis JR, Goodwin LA, Woyke T, Teshima H, Bruce D, Detter C, Tapia R, Han S, Han J, Pitluck S Sello JK Genome sequence of Amycolatopsis sp. strain ATCC 39116, a plant biomass-degrading actinomycete. J Bacteriol 194:2396-2397 (2012**)**
[8] Di Gioia D, Luziatelli F, Negroni A, Ficca AG, Fava F, Ruzzi M Metabolic engineering of Pseudomonas fluorescens for the production of vanillin from ferulic acid. J Biotechnol 156:309-316 (2011**)**
[9] European Commission (EC) Regulation No 1334/2008 of the european parliament and of the council of 16 december 2008 on flavourings and certain food ingredients with flavouring properties for use in and on foods and amending regulation (EC) no 1601/91 of the council, regulations (EC) no 2232/96 and (EC) no 110/2008 and directive 2000/13/EC.
[10] Fleige C, Hansen G, Kroll J, Steinbuchel A Investigation of the Amycolatopsis sp. strain ATCC 39116 vanillin dehydrogenase and its impact on the biotechnical production of vanillin. Appl Environ Microbiol 79:81-90 (2013**)**
[11] *Fleige C, Hansen G, Kroll J, Steinbüchel A, Lambrecht S, Pesaro M, Hilmer J* Microorganisms and methods for producing substituted phenols.
   EP 0761817 A1
[12] Jefferson RA, Burgess SM, Hirsh D Beta-glucuronidase from Escherichia coli as a gene-fusion marker. Proc Natl Acad Sci USA 83:8447-8451 (1986**)**
[13] Laemmli UK Cleavage of structural proteins during the assembly of the head of bacteriophage T4. Nature 227:680-685 (1976**)**
[14] Lal R, Khanna R, Dhingra N, Khanna M, Lal S Development of an improved cloning vector and transformation system in Amycolatopsis mediterranei (Nocardia mediterranei). J Antibiot 51:161-169 (1998**)**
[15] Muheim A, Lerch K Towards a high-yield bioconversion of ferulic acid to vanillin. Appl Microbiol Biotechnol 51:456-461 (1999**)**
[16] Myronovskyi M, Welle E, Fedorenko V, Luzhetskyy A Beta-glucuronidase as a sensitive and versatile reporter in actinomycetes. Appl Environ Microbiol 77:5370-5383 (2011**)**
[17] Olano C, Lombó F, Méndez C, Salas JA Improving production of bioactive secondary metabolites in actinomycetes by metabolic engineering. Metab Eng 10:281-292 (2008**)**
[18] Overhage J, Priefert H, Rabenhorst J, Steinbüchel A Biotransformation of eugenol to vanillin by a mutant of Pseudomonas sp. strain HR199 constructed by disruption of the vanillin dehydrogenase (vdh) gene. Appl Microbiol Biotechnol 52:820-828 (1999**)**
[19] Overhage J, Steinbüchel A, Priefert H Harnessing eugenol as a substrate for production of aromatic compounds with recombinant strains of Amycolatopsis sp. HR167. J Biotechnol 125:369-376 (2006**)**
[20] Pickens LB, Tang Y, Chooi Y Metabolic engineering for the production of natural products. Annu Rev Chem Biomol Eng 2:211-236 (2011**)**
[21] Plaggenborg R, Overhage J, Loos A, Archer JAC, Lessard P, Sinskey AJ, Steinbüchel A Priefert H Potential of Rhodococcus strains for biotechnological vanillin production from ferulic acid and eugenol. Appl Microbiol Biotechnol 72:745-755 (2006**)**
[22] Power P, Dunne T, Murphy B, Lochlainn LN, Rai D, Borissow C, Rawlings B, Caffrey P Engineered synthesis of 7-oxo- and 15-deoxy-15-oxo-amphotericins: Insights into structure-activity relationships in polyene antibiotics. Chem Biol 15:78-86 (2008**)**
[23] Priefert H, Achterholt S, Steinbüchel A Transformation of the pseudonocardiaceae Amycolatopsis sp. strain HR167 is highly dependent on the physiological state of the cells. Appl Microbiol Biotechnol 58:454-460 (2002**)**
[24] Priefert H, Rabenhorst J, Steinbüchel A Biotechnological production of vanillin. Appl Microbiol Biotechnol 56:296-314 (2001**)**
[25] Rowe CJ, Cortes J, Gaisser S, Staunton J, Leadlay PF Construction of new vectors for high-level expression in actinomycetes. Gene 216:215-223 (1998**)**
[26] Sambrook J, Fritsch EF, Maniatis T Molecular cloning: A laboratory manual. 2nd edition. Cold Spring Harbor, New York.(1998**)**
[27] Tuteja D, Dua M, Khanna R, Dhingra N, Khanna M, Kaur H, Saxena DM, Lal R The importance of homologous recombination in the generation of large deletions in hybrid plasmids in Amycolatopsis mediterranei. Plasmid 43:1-11 (2000**)**
[28] Weber K, Osborn M The reliability of molecular weight determinations by dodecyl sulfate-polyacrylamide gel electrophoresis. J Biol Chem 244:4406-4412 (1969**)**
[29] Wilkinson CJ, Hughes-Thomas ZA, Martin CJ, Bohm I, Mironenko T, Deacon M, Wheatcroft M, Wirtz G, Staunton J, Leadlay PF Increasing the efficiency of heterologous promoters in actinomycetes. J Mol Microbiol Biotechnol 4:417-426 (2002**)**

### EXAMPLES

### Example 1

### Construction of expression vectors

In order to construct a plasmid-based expression system for *Amycolatopsis* sp. ATCC 39116, the shuttle vector p6apra, conferring resistance to apramycin, was generated.

**Figure 1** provides a physical map of the new shuttle vector p6apra. ApraR: apramycin resistance cassette; pA-rep: origin of replication for *Amycolatopsis* from plasmid pA387; *oriV:* origin of replication for *E. coli* from plasmid pBR322. Unique restriction sites, which can be used for cloning, are presented

The former *E. coli- Amycolatopsis* shuttle vector pRLE6 was modified because we observed recombination events between the included kanamycin resistance gene and an inactive genomic copy of the *npt* gene. Furthermore, a different antibiotic resistance cassette was necessary since we previously generated a kanamycin resistant deletion mutant of *Amycolatopsis* sp. ATCC 39116 that should serve as host as well. For this purpose we removed the *npt* gene of pRLE6 by digestion with Smal and inserted the apramycin resistance cassette of the transposon pMA5096 by blunt end ligation into the vector backbone. After the exchange of the antibiotic resistance gene, the vector was transferred to *Amycolatopsis* sp. ATCC 39116 and apramycin resistant mutants could be monitored. We were able to isolate plasmid DNA from each tested clone, which suggests that p6apra is maintained as an autonomous element. Furthermore, the vector replicates in a stable manner as no deletions or rearrangements were observed after isolation and analysis of the plasmid from the obtained mutants.

Based on the new shuttle vector, four derivatives with different promoters were generated. All promoter fragments were cloned into the single EcoRV site antilinear to the apramycin resistance cassette, respectively. As a result of this insertion at least five of the remaining unique restriction sites upstream of each promoter are available for cloning and expression of foreign DNA under control of these regulatory elements.

**Figure 2** provides physical maps of the generated expression vectors. ApraR: apramycin resistance cassette; pA-rep: origin of replication for *Amycolatopsis* from plasmid pA387; *oriV:* origin of replication for *E. coli* from plasmid pBR322. Integrated promoters and their directions are indicated by grey arrows. Unique restriction sites, which can be used for cloning, are presented.

To test different promoters in *Amycolatopsis* sp. ATCC 39116 we first chose the commonly used *lac*-promoter, which is reported to be functional in a broad range of bacteria and which is therefore used within various cloning vectors. To obtain additional restriction sites, we inserted the complete multiple cloning site of the commercially available plasmid pBluescript SK- into p6apra. For this the whole multiple cloning site including the *lac*-promoter was amplified by PCR and subsequently cloned into the EcoRV site of p6apra.

As a second promoter, we inserted the upstream region of the previously characterized *vdh* gene of *Amycolatopsis* sp. ATCC 39116. For this purpose we amplified a 395-bp fragment upstream of the start codon of *vdh* (GTG) that contains the promoter sequence as was shown in complementation experiments beforehand (data not shown). To maintain this functional genetic organization, we inserted a Ncol-site (CCATGG) in the reverse primer, which allows us to clone a gene of interest 6bp downstream of the putative Shine-Dalgarno sequence of *vdh* (AGGAG).

The same cloning strategy was used for the insertion of the promoter region of the kanamycin resistance gene (Km^{r}). Analogous to the *vdh*-promoter we maintained a distance of 9 bp to the native Shine-Dalgarno sequence of the *npt* gene by introduction of a Ncol site. The kanamycin resistance promoter was chosen since we knew from the use of pRLE6 and Km^{r} for the introduction of knock outs that *npt* is functionally transcribed in *Amycolatopsis* sp. ATCC 39116 under the control of this element.

Finally, we cloned the *ermE** promoter from *Saccharopolyspora erythraea,* which is a strong constitutive promoter and which is frequently used in actinomycetes if high-level expression is required (Wilkinson et al. 2002). The promoter was removed from the plasmid pIAGO by KpnI/BamHI digestion. Blunt ends were subsequently generated, and the fragment was again cloned into the EcoRV-site of p6apra.

All generated plasmids replicated in *Amycolatopsis* sp. ATCC 39116 without any observable rearrangements, and they can therefore be used for the heterologous expression of required genes.

### Example 2

### Expression of gusA in Amycolatopsis sp. A TCC 39116

In order to test the applicability of the heterologous promoters in *Amycolatopsis* sp. ATCC 39116, the β-glucuronidase GUS was chosen, encoded by the *gusA* gene as a reporter [16]. The activity of the β-glucuronidase can easily be detected on agar plates qualitatively and also quantitatively in a simple but sensitive spectrophotometric assay.

Initially *Amycolatopsis* sp. ATCC 39116 was tested for an inherent GUS activity to exclude a potential background signal in the control strains. The wild type strain did not exhibit any glucuronidase activity, which makes *gusA* a suitable reporter gene for our investigation.

To determine the functionality of the expression plasmids, the glucuronidase gene *gusA* was cloned as a reporter downstream and collinear to the promoters, respectively. As a control strain we additionally generated a promoterless version of p6apra containing only *gusA.*

After the transfer of the expression vectors, GUS activity was detected on agar plates for all strains except for the control strain that carried the promoterless *gusA* gene. For this purpose, the bacteria were incubated for 48 hours at 42°C on solid TSB agar and subsequently overlaid with the substrate X-Gluc as described in materials and methods. Differences in the expression strength of the cloned promoter fragments could be assumed even within this qualitative analysis. The expression strains carrying p6plac::*gusA* and p6pvdh::*gusA* exhibited only a faint blue color, while a strong coloring was observed with strains *Amycolatopsis* sp. ATCC 39116 p6pKm::*gusA* and *Amycolatopsis* sp. ATCC 39116 p6permE::*gusA*.

To further analyze the *gusA* expression under the control of the different promoters, the specific enzyme activity was determined in crude extracts of the four expression strains. The different *Amycolatopsis* sp. ATCC 39116 expression strains were cultivated in TSB medium for 24 h at 42 °C. The cells were harvested after cultivation, soluble fractions were prepared, and the specific activity of the β-glucuronidase GUS was measured photometrically as described in materials and methods. The control strain carrying the promoterless plasmid p6apra::*gusA* was treated under the same conditions.

The activity assays revealed that no GUS activity was expressed by the control strain. In contrast to that all generated expression strains showed the formation of p-nitrophenol as observed spectrophotometrically by the increase of absorption at 415 nm. Quantitative analysis confirmed the assumption of an increasing expression strength with p6permE exhibiting the highest enzyme activity **(Table 2).** This was also obvious during the activity tests on agar plates. While *gusA* expression under the control of the *lac* and *vdh* promoter only led to an enzyme activity of 0.04 U/mg ± 0.00038 U/mg and 4.04 U/mg ± 0.071 U/mg, respectively, the strains carrying the Km^{r}-promoter and *PermE** exhibited very strong activities of 59.07 U/mg ± 1.50 U/mg and 302.20 U/mg ± 1.25 U/mg.

The expression of *gusA* was also obvious during SDS-PAGE analysis of the soluble fractions which revealed a prominent protein band at a size of approximately 69 ± 2 kDa. This signal was missing for the control strain, while the strongest protein band could be observed for the strain expressing *gusA* under the control of *PermE*.*

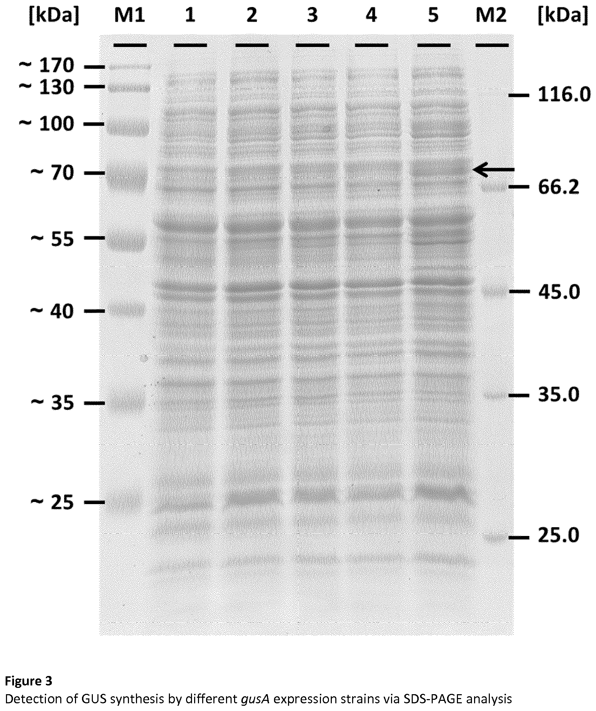

**Figure 3** shows the detection of GUS synthesis by different *gusA* expression strains via SDS-PAGE analysis. The samples were separated in a 12.5 % (wt/vol) SDS-polyacrylamide gel as described in materials and methods. The gel was stained with Serva Blue G. Lane
**M1:** Molecular weight marker: "PageRuler Prestained Protein Ladder", Lane
**M2:** Molecular weight marker: "Unstained Protein Molecular Weight Marker"; Lane
**1:** 30 pig protein of the soluble fraction of the control strain *Amycolatopsis* sp. ATCC 39116 p6apra::*gusA*
**2:** 30 µg protein of the soluble fraction of *Amycolatopsis* sp. ATCC 39116 p6plac::*gusA*
**3:** 30 µg protein of the soluble fraction of *Amycolatopsis* sp. ATCC 39116 p6pvdh::*gusA*
**4:** 30 µg protein of the soluble fraction of *Amycolatopsis* sp. ATCC 39116 p6pKm::*gusA*
**5:** 30 µg protein of the soluble fraction of *Amycolatopsis* sp. ATCC 39116 p6permE::gusA.

Cells were grown in TSB medium for 24 h. Soluble fractions were obtained after cell disruption and subsequent centrifugation as described in materials and methods. The arrow indicates the corresponding signal of the putative *gusA* gene product.

In the following **Tables 1 and 2** provide Bacterial strains and plasmids as well as an overview of the specific GUS activity of the different expression strains.

**Table 1**

| Bacterial strains and plasmids used in the study | | |
|---|---|---|
| **Bacterial strains and plasmids** | **Description, sequence** | **Reference** |
| **Strains** | | |
| *Amycolatopsis* sp. ATCC 39116 | wild type | American Type Culture Collection, No. 39116 |
| *E. coli* Mach-1 T1 | F⁻ φ80(*lacZ*)ΔM15 Δ*lacX*74 *hsd*R (r_{K}⁻ m_{K}⁺) Δ*recA*1398 *endA1 tonA* | Invitrogen GmbH (Karlsruhe, Germany) |
| *E. coli* K12 ER 2925 | *ara-14 leu86 fhuA31 aclY1 tsx78 glnV44 galK2 galT22 mcrA dcm-6 hisG4 rfbD1 R*(*zgb210*::*Tn10*)*TetS endA1 rpsL136 dam13*::*Tn9 xylA-5 mtl-1 thi-1 mcrB1 hsdR2* | New England Biolabs Inc. (Ipswich, MA, USA) |

| **Plasmids** | | |
|---|---|---|
| pRLE6 | 5.8 kb, Km^{r} | (Priefert et al. 2002) |
| pBluescript SK-(pSK-) | Ap^{r}, *LacPOZ',* T7 and T3-promoter | Agilent GmbH, Waldbronn, Germany |
| pIAGO | *ermE**-promoter from *Saccharopolyspora erythraea* as 0.28-kb KpnI-BamHI fragment, Ts^{r}, Ap^{r} | (Power et al. 2008) |
| pMA5096 | Ap^{r}; Apra^{r} transposable plasmid comprising transposase Tn5096; | (Banh et al. 2005) |
| pSETGUS | *Streptomyces* integrative vector, *gusA* under control of *tipA*-promoter | (Myronovskyi et al. 2011) |
| p6apra | pRLE6 with Apra^{r} from pMA5096 cloned into SmaI-sites | This invention |

| **Bacterial strains and plasmids** | **Description, sequence** | **Reference** |
|---|---|---|
| p6permE | p6apra with *ermE**-promoter from pIAGO cloned into EcoRV-site | This invention |
| p6pKm | p6apra with Km^{r}-promoter from pRLE6 cloned into EcoRV-site | This invention |
| p6pvdh | p6apra with promoter region of *vdh*-gene from *Amycolatopsis* sp. ATCC 39116 (Fleige et al. 2013) cloned into EcoRV-site | This invention |
| p6plac | p6apra with *multiple cloning site* of pSK-cloned into EcoRV-site | This invention |
| p6apra::*gusA* | Spel/EcoRV fragment of pSETGUS containing *gusA* cloned into Nhel/Dral sites of p6apra | This invention |
| p6permE::*gusA* | Spel/EcoRV fragment of pSETGUS containing *gusA* cloned into Nhel/Dral sites of p6permE | This invention |
| p6pKm::*gusA* | Spel/EcoRV fragment of pSETGUS containing *gusA* cloned into Nhel/Dral sites of p6pKm | This invention |
| p6pvdh::*gusA* | Spel/EcoRV fragment of pSETGUS containing *gusA* cloned into Nhel/Dral sites of p6pvdh | This invention |
| p6plac::*gusA* | Spel/EcoRV fragment of pSETGUS containing *gusA* cloned into Spel/EcoRV-sites of p6plac | This invention |

| **Oligonucleotides** | **Sequence 5' - 3'** | |
|---|---|---|
| Apra_for | GTAGCGGGGATCCGGGGA | This invention |
| Apra rev | GCTCGGTTCATGTGCAGCTCC | This invention |
| pSK_MCS_for | GCCGATTTCGGCCTATTG | This invention |
| pSK_MCS_rev | GCGAGTCAGTGAGCGAGG | This invention |
| pKm_for | GCAAGCGAACCGGAATTG | This invention |
| pKm_Ncol_rev | CAACCATGGGAAACGATCCTCATCCTGTCT | This invention |
| pvdh_for | CCAGATGCGTGTACGGGTG | This invention |
| pvdh_Ncol_rev. | AGCCATGGGTCACTCCTGATGCG | This invention |

**Table 2**

| **Specific GUS activity of the different expression strains** | |
|---|---|
| **Expression strain** | **Specific GUS activity (U/mg)** |
| *Amycolatopsis* sp. ATCC 39116 p6plac::*gusA* | 0.04 U/mg ± 0.0004 U/mg |
| *Amycolatopsis* sp. ATCC 39116 p6pvdh::*gusA* | 4.04 U/mg ± 0.071 U/mg |
| *Amycolatopsis* sp. ATCC 39116 p6pKm::*gusA* | 59.07 U/mg ± 1.50 U/mg |
| *Amycolatopsis* sp. ATCC 39116 p6permE::*gusA* | 302.20 U/mg ± 1.25 U/mg |

## Claims

1. A shuttle vector, designated p6apra, with resistance to apramycin obtainable in that shuttle vector pRLE6 of *E.coli-Amycolatopsis*
(a) is subjected to digestion with a suitable endonuclease (Smal, Xmal) to remove the *npt* gene from said vector or the vector backbone is amplified by inverse PCR without the *npt* gene and
(b) the apramycin resistance gene *aac(3)VI* is inserted into the vector backbone by ligation.

2. A process for obtaining shuttle vector p6apra, wherein [] the shuttle vector pRLE6 of *E.coli-Amycolatopsis*
(a) is subjected to digestion with Smal to remove the *npt* gene from said vector and
(b) the apramycin resistance cassette of the transposon pMA5096 is inserted into the vector backbone by blunt end ligation.

3. The vector of Claim 1 wherein
(a) *lac*-promoter with the multiple cloning site of the plasmid pBluescript SK is amplified via PCR
(b) the PCR product is cloned into the EcoRV site of the p6apra vector according to Claim 1.

4. The process of Claim 2 wherein
(a) *lac*-promoter with the multiple cloning site of the plasmid pBluescript SK is amplified via PCR, and
(b) the PCR product is cloned into the EcoRV site of the p6apra vector according to Claim 1.

5. The vector of Claim 1 wherein
(a) a 395-bp fragment of the genome of *Amycolatopsis* sp. ATCC 39116 upstream of the start codon of *vdh* (GTG) containing a promoter sequence is amplified via PCR,
(b) a Ncol-site (CCATGG) is inserted into the reverse primer 6bp downstream of the Shine-Dalgarno sequence of *vdh* (AGGAG), and
(c) the PCR product is cloned into the EcoRV site of the p6apra vector according to Claim 1.

6. The process of Claim 2 wherein
(a) a 395-bp fragment gene of *Amycolatopsis* sp. ATCC 39116 upstream of the start codon of *vdh* (GTG) containing a promoter sequence is amplified via PCR,
(b) a Ncol-site (CCATGG) is inserted into the reverse primer 6bp downstream of the Shine-Dalgarno sequence of *vdh* (AGGAG), and
(c) the PCR product is cloned into the EcoRV site of the p6apra vector according to Claim 1.

7. The vector of Claim 1 wherein
(a) the promoter sequence of the kanamycin resistance gene (Km^{r}) is amplified via PCR,
(b) a Ncol-site (CCATGG) is inserted into the reverse primer 9bp downstream of the Shine-Dalgarno sequence of *npt,* and
(b) the PCR product is cloned into the EcoRV site of the p6apra vector according to Claim 1.

8. The process of Claim 2 wherein
(a) the promoter sequence of the kanamycin resistance gene (Km^{r}) is amplified via PCR,
(b) a Ncol-site (CCATGG) is inserted into the reverse primer 9bp downstream of the Shine-Dalgarno sequence of *npt,* and
(c) the PCR product is cloned into the EcoRV site of the p6apra vector according to Claim 1.

9. The vector of Claim 1 []wherein <
(a) ermE* promoter from *Saccharopolyspora erythraea* is removed from the plasmid pIAGO, and
(b) the fragment is cloned into the EcoRV site of the p6apra vector according to Claim 1.

10. The process of Claim 2 wherein
(a) ermE* promoter from *Saccharopolyspora erythraea* is removed from the plasmid pIAGO, and
(b) the fragment is cloned into the EcoRV site of the p6apra vector according to Claim 1.

11. A plasmid expression system for the order of Actinomycetales, containing several industrially relevant families, like Corynebacterineae, Pseudonocardineae or Streptomycineae comprising at least one of the expression vectors according to any of the Claims 1, 3, 5, 7 or 9.

12. The use of at least one of the expression vectors according to any of the Claims 1, 3, 5, 7 or 9 for replication in these bacteria.

13. The use of at least one of the expression vectors according to any of the Claims 1, 3, 5, 7 or 9 for heterologous expression of genes in these bacteria.

14. The use of recombinant bacteria containing at least one of the expression vectors according to any of the Claims 1, 3, 5, 7 or 9 in a process for the manufacture of Vanillin, Vanillic acid, Coniferylalkohol or Coniferylaldehyd.

15. A process for making nature-identic vanillin by fermentation of ferulic acid, or eugenol by means of microorganisms containing at least one of the expression vectors according to any of the Claims 1, 3, 5, 7 or 9.

## Patentansprüche

1. Shuttle-Vektor mit der Bezeichnung *p6apra* mit Resistenz gegen *Apramycin,* dadurch erhältlich, dass der Shuttle-Vektor *pRLE6* aus *Ecoli-Amycolatopsis,*
(a) einem Aufschluss mit einer geeigneten Endonuclease (*SmaI, XmaI*) unterworfen wird, um das *npt-*Gen von besagtem Vektor zu entfernen oder der Hauptstrang des Vektors durch inverse PCR ohne das *npt*-Gen amplifiziert wird; und
(b) das Apramycin-resistente Gen *aac(3)VI* durch Ligation in den Hauptstrang des Vektors eingebaut wird.

2. Verfahren zur Herstellung des Shuttle-Vektors p6apra wobei man den Shuttle-Vektor *pRLE6* aus *Ecoli-Amycolatopsis*
(a) einem Aufschluss mit *SmaI* unterwirft, um das *npt-Gen* von besagtem Vektor zu entfernen, und
(b) die Apramycin-resistente Kassette des Transposons *pMA5096* in den Hauptstrang des Vektors durch Abstumpfung und Ligation einbaut.

3. Vektor nach Anspruch 1, wobei
(a) der *lac*-Promoter mit der multipler Klonierungsseite des Plasmids *pBluescript SK* durch PCR amplifiziert wird, und
(b) das PCR-Produkt in die EcoRV Seite des *p6apra-*Vektors nach Anspruch 1 einkloniert wird.

4. Verfahren nach Anspruch 2, wobei
(a) der lac-Promoter mit der multiplen Klonierungsseite des Plasmids *pBluescript SK* durch PCR amplifiziert wird, und
(b) das PCR-Produkt in die EcoRV Seite des *p6apra*-Vektors nach Anspruch 1 einkloniert wird.

5. Vektor nach Anspruch 1, wobei
(a) ein 395-bp Fragment des Genoms von Amycolatopsis sp. ATCC 39116 oberhalb des Startcodons von *vdh* (GTG) enthaltend die Promoter-Sequenz durch PCR amplifiziert wird;
(b) eine *Ncol*-Position (CCATGG) in den reversen Primer *6bp* unterhalb der Shine-Delgarno-Sequenz von *vdh* (AGGAG) eingebaut wird; und
(c) das PCR-Produkt in die EcoRV Seite des *p6apra*-Vektors nach Anspruch 1 einkloniert wird.

6. Verfahren nach Anspruch 2, wobei
(a) ein 395-bp Fragment des Genoms von Amycolatopsis sp. ATCC 39116 oberhalb des Startcodons von *vdh* (GTG) enthaltend die Promoter-Sequenz durch PCR amplifiziert wird;
(b) eine *Ncol*-Position (CCATGG) in den reversen Primer *6bp* unterhalb der Shine-Delgarno-Sequenz von *vdh* (AGGAG) eingebaut wird; und
(c) das PCR-Produkt in die EcoRV Seite des *p6apra*-Vektors nach Anspruch 1 einkloniert wird.

7. Vektor nach Anspruch 1, wobei
(a) die Promoter-Sequenz des Kanamycin-resistenten Gens (*Km^{r}*) durch PCR amplifiziert wird;
(b) eine *Ncol*-Position (CCATGG) in den reversen Primer *9bp* unterhalb der Shine-Delgarno Sequenz von *npt* eingebaut wird; und
(c) das PCR-Produkt in die EcoRV Seite des *p6apra*-Vektors nach Anspruch 1 einkloniert wird.

8. Verfahren nach Anspruch 2, wobei
(a) die Promoter-Sequenz des Kanamycin-resistenten Gens (*Km^{r}*) durch PCR amplifiziert wird;
(b) eine *NcoI*-Position (CCATGG) in den reversen Primer *9bp* unterhalb der Shine-Delgarno Sequenz von *npt* eingebaut wird; und
(c) das PCR-Produkt in die EcoRV Seite des *p6apra*-Vektors nach Anspruch 1 einkloniert wird.

9. Vektor nach Anspruch 1, wobei
(a) der *ermE** Promoter von *Saccharopolyspora erythraea* aus dem Plasmid *pIAGO* entfernt wird; und
(b) das Fragment in die EcoRV Seite des *p6apra*-Vektors nach Anspruch 1 einkloniert wird.

10. Verfahren nach Anspruch 2, wobei
(a) der *ermE** Promoter von *Saccharopolyspora erythraea* aus dem Plasmid *pIAGO* entfernt wird; und
(b) das Fragment in die EcoRV Seite des *p6apra*-Vektors nach Anspruch 1 einkloniert wird.

11. Plasmid-Expressionssystem für die Ordnung Actinomycetales, enthaltend verschiedene industriell relevante Familien, wie *Corynebacterineae, Pseudonocardineae* oder *Streptomycineae* enthaltend wenigstens einen Expressionsvektor nach einem der Ansprüche 1,3,5,7 oder 9.

12. Verwendung wenigstens eines Expressionsvektors nach einem der Ansprüche 1, 3, 5, 7 oder 9 für die Replizierung von Bakterien.

13. Verwendung wenigstens eines Expressionsvektors nach einem der Ansprüche 1, 3, 5, 7 oder 9 für die heterologe Expression von Genen in diesen Bakterien.

14. Verwendung von rekombinanten Bakterien enthaltend wenigstens einen der Expressionsvektoren nach den Ansprüchen 1, 3, 5, 7 oder 9 in einem Verfahren zur Herstellung von Vanillin, Vanillinsäure, Coniferylalkohol oder Coniferylaldehyd.

15. Verfahren zur Herstellung natur-identischen Vanillins durch Fermentierung von Ferulasäure oder Eugenol mit Hilfe von Mikroorganismen enthaltend mindestens einen Expressionsvektor nach einem der Ansprüche 1, 3, 5, 7 oder 9.

## Revendications

1. Vecteur-navette, désigné p6apra, avec une résistance à l'apramycine que l'on peut obtenir en ce que le vecteur-navette pRLE6 d'*E*. *coli-Amycolatopsis*
(a) est soumis à une digestion avec une endonucléase appropriée (SmaI, XmaI) afin d'ôter le gène *npt* dudit vecteur, ou le squelette du vecteur est amplifié par une ACP inverse sans le gène *npt,* et
(b) le gène *aac(3)VI* de résistance à l'apramycine est inséré dans le squelette du vecteur par ligature.

2. Processus destiné à obtenir un vecteur-navette p6apra, dans lequel le vecteur-navette pRLE6 d'E. *coli-Amycolatopsis*
(a) est soumis à une digestion avec Smal afin d'ôter le gène *npt* dudit vecteur, et
(b) la cassette de résistance à l'apramycine du transposon pMA5096 est insérée dans le squelette du vecteur par une ligature à extrémités franches.

3. Vecteur selon la revendication 1, dans lequel
(a) le promoteur *lac,* avec le site de clonage multiple du plasmide pBluescript SK, est amplifié via une ACP, et
(b) le produit de l'ACP est cloné dans le site EcoRV du vecteur p6apra, selon la revendication 1.

4. Processus selon la revendication 2, dans lequel
(a) le promoteur *lac,* avec le site de clonage multiple du plasmide pBluescript SK, est amplifié via une ACP, et
(b) le produit de l'ACP est cloné dans le site EcoRV du vecteur p6apra, selon la revendication 1.

5. Vecteur selon la revendication 1, dans lequel
(a) un fragment de 395 pb du génome *d'Amycolatopsis* sp. ATCC 39116, en amont du codon d'initiation du *vdh* (GTG) contenant une séquence de promoteur, est amplifié via une ACP,
(b) un site Ncol (CCATGG) est inséré dans l'amorce arrière à 6pb en aval de la séquence de Shine-Dalgarno du *vdh* (AGGAG), et
(c) le produit de l'ACP est cloné dans le site EcoRV du vecteur p6apra, selon la revendication 1.

6. Processus selon la revendication 2, dans lequel
(a) un gène à fragment de 395 pb *d'Amycolatopsis* sp. ATCC 39116, en amont du codon d'initiation du *vdh* (GTG) contenant une séquence de promoteur, est amplifié via une ACP,
(b) un site Ncol (CCATGG) est inséré dans l'amorce arrière à 6pb en aval de la séquence de Shine-Dalgarno du *vdh* (AGGAG), et
(c) le produit de l'ACP est cloné dans le site EcoRV du vecteur p6apra, selon la revendication 1.

7. Vecteur selon la revendication 1, dans lequel
(a) la séquence de promoteur du gène de résistance à la kanamycine (Km^{r}) est amplifiée via une ACP,
(b) un site Ncol (CCATGG) est inséré dans l'amorce arrière à 9pb en aval de la séquence de Shine-Dalgarno du *npt,* et
(c) le produit de l'ACP est cloné dans le site EcoRV du vecteur p6apra, selon la revendication 1.

8. Processus selon la revendication 2, dans lequel
(a) la séquence de promoteur du gène de résistance à la kanamycine (Km^{r}) est amplifiée via une ACP,
(b) un site Ncol (CCATGG) est inséré dans l'amorce arrière à 9pb en aval de la séquence de Shine-Dalgarno du *npt,* et
(c) le produit de l'ACP est cloné dans le site EcoRV du vecteur p6apra, selon la revendication 1.

9. Vecteur selon la revendication 1, dans lequel
(a) le promoteur ermE* provenant de *Saccharopolyspora erythraea* est ôté du plasmide pIAGO, et
(b) le fragment est cloné dans le site EcoRV du vecteur p6apra, selon la revendication 1.

10. Processus selon la revendication 2, dans lequel
(a) le promoteur ermE* provenant de *Saccharopolyspora erythraea* est ôté du plasmide pIAGO, et
(b) le fragment est cloné dans le site EcoRV du vecteur p6apra, selon la revendication 1.

11. Système d'expression plasmidique pour l'ordre des Actinomycétales, contenant plusieurs familles pertinentes pour l'industrie comme les Corynebacterineae, Pseudonocardineae ou Streptomycineae, comprenant au moins l'un des vecteurs d'expression selon l'une quelconque des revendications 1, 3, 5, 7 ou 9.

12. Utilisation d'au moins l'un des vecteurs d'expression, selon l'une quelconque des revendications 1, 3, 5, 7 ou 9, pour une réplication dans ces bactéries.

13. Utilisation d'au moins l'un des vecteurs d'expression, selon l'une quelconque des revendications 1, 3, 5, 7 ou 9, pour une expression hétérologue de gènes dans ces bactéries.

14. Utilisation de bactéries recombinantes contenant au moins l'un des vecteurs d'expression, selon l'une quelconque des revendications 1, 3, 5, 7 ou 9, dans un processus destiné à la fabrication de vanilline, d'acide vanillique, d'un alcool coniférylique ou d'un aldéhyde coniférylique.

15. Processus d'élaboration d'une vanilline identique au produit naturel, par fermentation d'acide férulique ou d'eugénol, au moyen de microorganismes contenant au moins l'un des vecteurs d'expression selon l'une quelconque des revendications 1, 3, 5, 7 ou 9.
